# DEMANDE DE BREVET EUROPEEN

(11) **EP 2 108 659 A1**
(43) Date de publication de la demande: **14.10.2009**
(21) Numéro de dépôt: 09166591.9
(22) Date de dépôt: 25.10.2004
(51) Int. Cl.: C07K 14/755, A61K 38/37, C07K 1/34, G01N 33/68

(54) **Facteur VIII viralement sécurisé à faible teneur en multimères supérieurs**

(30) Priorité: 23.10.2003 FR 0312398
(62) Demande divisionnaire de: 04805296.3
(71) Demandeur: Laboratoire Français du Fractionnement et des Biotechnologies, 91940 Les Ulis (FR)
(72) Inventeur: Chtourou, Abdessatar (Sami), 78990 Elancourt (FR); Nogre, Michel, 92170 Vanves (FR); Schmitthaeusler, Roland, 78180 Guyancourt (FR)
(74) Mandataire: Warcoin, Jacques

(57) **Abrégé**

La présente invention se rapporte à une composition de Facteur VIII d'origine plasmatique sécurisé viralement, obtenue après filtration nanométrique, comprenant du Facteur von Willebrand (FvW) à un taux inférieur ou égal à 15 % de décamères et multimères supérieurs. De telles compositions présentent un facteur de réduction du titre des virus supérieur à 4 log et sont donc adaptées pour le traitement de l'hémophilie.

## Description

La présente invention se rapporte à une composition de Facteur VIII d'origine plasmatique sécurisée viralement, obtenue après filtration nanométrique, comprenant du Facteur von Willebrand (FvW) à un taux inférieur ou égal à 15 % de décamères et multimères supérieurs. De telles compositions présentent un facteur de réduction du titre des virus supérieur à 4 log et sont donc adaptées pour le traitement de l'hémophilie.

Le disponibilité en facteur de coagulation est en depuis un certain temps un problème de santé public. Pour répondre à la demande, les industriels ont développés des techniques de production de facteurs recombinants dont on pensait qu'elles pourraient à terme supplanter la fabrication à partir de pool de plasma. Toutefois, les quantités produites apparaissent encore insatisfaisante et les investissements pour le développement de ces produits sont conséquents. Par ailleurs, une réaction immunitaire contre ces facteurs recombinants est observée chez les patients, ce qui implique l'administration de dose élevée pour aboutir à l'effet thérapeutique souhaité. Enfin, certains patients ne tolèrent pas les facteurs recombinants.

Ainsi, la production de facteurs de coagulation d'origine plasmatique reste un enjeu important.

Le facteur VIII ou facteur anti-hémophilique est une protéine plasmatique présente en faible concentration dans le plasma humain. Ce facteur catalyse les réactions biochimiques de la coagulation du sang par augmentation de la vitesse de réaction pour aboutir à la formation d'un caillot de fibrine hémostatique obtenu à partir du fibrinogène soluble soumis à l'action de la thrombine en présence de calcium. Le facteur VIII intervient dans la cascade de réactions aboutissant à la thrombinoformation qui est l'activité enzymatique responsable de la transformation du fibrinogène en fibrine. Le point central de la coagulation repose donc sur la présence et l'activation du FVIII.

Les individus hémophiles, déficients pour le FVIII, sont traités par injection de ces FVIII purifiés obtenus soit par recombinaison génétique, soit par extraction du plasma humain.

Dans ce dernier cas, des procédés d'inactivation et/ou d'élimination de virus doivent être appliqués afin de protéger les hémophiles soignés par ces concentrés de toute infection due à des virus transmissibles par le sang ou ses dérivés : virus des hépatites A, B, C, VIH ou parovirus B19....

Ainsi, un des problèmes essentiels liés à la préparation du facteur VIII à partir du plasma, réside dans la nécessité d'inactiver et/ou d'éliminer des virus originellement contenus dans le sang au minimum selon les normes réglementaires tout en conservant un rendement optimum en facteur à l'issue du procédé. De nombreuses techniques d'inactivation virale ont ainsi été développées telles que le chauffage à sec, la pasteurisation, le traitement solvant-détergent. L'ensemble de ces techniques est relativement efficace vis-à-vis des virus enveloppés mais l'inactivation ou l'élimination des virus nus, notamment des petits virus tels que le parvovirus B19 ou le virus de l'hépatite A, constitue un obstacle majeur.

Des technologies plus récentes utilisent les capacités de rétention virale de membranes de faible taille de pores. Ces technologies présentent effectivement une efficacité notable vis-à-vis de virus de petite taille tels que les parvovirus B19 ou le virus de l'hépatite A et peuvent être appliquées à des protéines de faible poids moléculaire. Cependant, les seuils de coupure utilisés, qui sont inférieurs à 900 kD, ne permettent pas d'envisager la filtration de protéines ou complexes protéiques de haut poids moléculaire comme le facteur VIII sans perte majeure de rendement.

Le facteur VIII se présente comme un édifice protéique complexe d'une protéine active, le FVIII, transportée par une protéine de haut-poids moléculaire à laquelle le FVIII est lié par des liaisons ioniques et hydrophobes. La protéine de haut poids moléculaire est le Facteur von Willebrand (FvW) constitué d'un ensemble de monomères élémentaires ayant un degré de multimérisation variable conduisant à des structures assemblées en tétramère jusqu'à des édifices comportant plus de seize monomères.

Selon les méthodes de purification de FVIII mises en oeuvre, le produit final peut contenir du FvW à divers degré de multimérisation (METZNER, HERMENTIN et al. - Haemophilia (1998), 4 (Suppl. 3), 25-32).

Or, dans notre brevet FR 97 15888, nous avons décrit qu'il est possible de filtrer le FVIII plasmatique, malgré sa taille, tout en retenant des virus de taille supérieure ou égale à 20 nm, sur des filtres de porosité d'environ 15 nm avec une concentration en ions chaotropiques d'au moins 0,2 M.

Plus récemment, la recherche conduite pour perfectionner ce procédé et choisir différentes natures de matériaux filtrants a fait apparaître que la taille des pores du filtre et les limites techniques peuvent varier selon les fabricants. Ainsi, il est apparu nécessaire de trouver un test rapide et reproductible permettant de vérifier que le produit final répond bien aux exigences sanitaires.

L'assurance de l'élimination des virus par le moyen de filtres est garantie par des méthodes de validation effectuées sur le filtre après passage de solution de FVIII. Ces méthodes peuvent être par exemple la mesure de diffusion gazeuse à travers la membrane ou, dans le cas de filtres en curophane, la mesure du passage de particules d'or colloïdal calibrées à travers le filtre.

Mais aucune méthode ne se réfère au produit filtré lui-même pour déterminer qu'il a subi une filtration capable de retenir les virus dans des proportions fixées.

Une analyse plus fine de la composition des multimères du FVIII avant et après filtration a été réalisée, en même temps qu'une mesure de la réduction du titre viral apportée par la filtration de facteur VIII.

Nous avons trouvé de façon surprenante et inattendue que l'appauvrissement en multimères de haut poids moléculaire de FvW mesurés dans les filtrats de FVIII est corrélé avec l'efficacité de la rétention de virus par le filtre. En outre, nous avons découvert qu'il est possible de filtrer à environ 20 nm grâce à la vérification de la teneur en multimères. Nous proposons donc un nouveau moyen permettant la production à haut rendement et la caractérisation du FVIII qui répondent aux exigences d'élimination de virus par le filtre nanométrique.

### Description

Ainsi, dans un premier aspect, la présente invention porte sur une composition pharmaceutique de facteur VIII d'origine plasmatique dont la sécurité virale correspond à un facteur de réduction supérieur à 4 log, ce qui répond aux exigences de sécurité en matière d'élimination de virus par filtration. La composition de FVIII ainsi sécurisée est caractérisée par un faible taux résiduel de FvW de haute multimérisation.

Plus spécifiquement, l'invention concerne une composition de Facteur VIII d'origine plasmatique, obtenue après filtration à travers un filtre nanométrique d'ouverture nominale de pores de 15±2 nm à 23±2 nm, **caractérisée en ce qu**'elle comprend du Facteur von Willebrand (FvW) à un taux inférieur ou égal à 15 % de décamères et multimères supérieurs. Dans cette composition, le facteur de réduction du titre d'un virus d'une taille de 27±3 nm est supérieur ou égale à 4 log, de préférence 5 log, avantageusement 6 log comparé à la solution avant filtration.

Cette composition peut se trouver sous la forme d'une solution injectable par voie intraveineuse, intramusculaire ou sous-cutanée par exemple.

L'invention concerne également la corrélation entre la présence de 15 % au plus de décamères et multimères supérieurs de FvW avec un facteur de réduction du titre viral d'au moins 4 log.

Ainsi, dans un deuxième aspect, l'invention porte sur un procédé pour tester la sécurité virale d'une composition de Facteur VIII d'origine plasmatique, ledit procédé comprenant une étape consistant en la détermination du taux résiduel de FvW de haute multimérisation. En particulier, on considérera qu'une composition est sécurisée viralement dans le cas où l'on détecte moins de 15% de décamères et multimères supérieurs de FvW.

Dans un aspect complémentaire, l'invention porte sur un kit de test permettant de mettre en oeuvre le procédé mentionné ci-dessus comprenant les réactifs nécessaires pour le dosage des multimères de FvW de degré de multimérisation supérieur ou égal à 10.

L'invention porte également sur un procédé de préparation d'une solution de facteur VIII sécurisée viralement comprenant une étape de filtration à travers des filtres nanométriques d'ouverture nominale de pores comprise entre 15±2 nm et 23±2 nm, soit un intervalle de 13 nm à 25 nm, une étape de dosage du Facteur von Willebrand (FvW) de décamères et multimères supérieurs. L'étape de dosage consiste de préférence en une vérification que le taux de décamères et multimères supérieurs de FvW est inférieur ou égal à 15 %. On obtient à l'issue de ce procédé des solutions de facteur VIII dont le facteur de réduction du titre d'un virus d'une taille de 27±3 nm est supérieur ou égal à 4 log, de préférence 5 log, avantageusement 6 log. La solution de facteur VIII avant filtration comprend de manière optimale un ion chaotropique, par exemple CaCl2, à une concentration supérieure ou égale à 0.2 M, par exemple 0.25 ou 0.35 M.

L'invention vise également l'utilisation d'une composition mentionnée ci-dessus pour préparer un médicament destiné au traitement des maladies liées à la coagulation sanguine, notamment l'hémophilie.

### Exemple 1: Procédé de préparation de FVIII sécurisé par filtration sur filtre nanométrique de 15 nm et vérification de la teneur en FvW de multimérisation > 10.

Les virus testés sont des bactériophages Phi X 174, virus non enveloppés, de diamètre 27 ± 3 nm.
La culture des virus et leur titrage sont effectués conformément à la norme AFNOR : NF T 72-181.
Le FVIII est recueilli à la sortie de la colonne Toyopearl DEAE et amené à pH 6 ; la concentration en CaCl2 est portée à 0.35 M. La température de la solution et du filtre est thermostatée à 35 °C et la pression de filtration est ajustée à moins de 100 mbar.
Dans ces conditions, le débit est de 1.21/h par m2.

Le rendement en FVIII : C est d'environ 70 % par rapport au FVIII : C avant filtration.
Le tableau I donne la répartition des multimères de FvW.

**Tableau I: Répartition des multimères (Planova® 15N)**

| **Facteur vW** | **Avant filtre** | **Après filtre** |
|---|---|---|
| <pentamères | 41 % | 53 % |
| 5 à 9 mères | 34 % | 34 % |
| 10 à 15 mères | 15 % | **9 %** |
| 16 mères et + | 11 % | **4 %** |

On constate une diminution significative des décamères/pentadécamères : la répartition passe de 15% à 9%.

Pour les hexadecamères et plus, la réduction est encore plus importante : elle passe de 11 %à4%.

Le titrage des virus PhiX174 démontre une réduction de 6 log à la filtration.

### Exemple 2: Procédé de préparation de FVIII sécurisé par filtration sur filtre nanométrique de 20 nm et vérification de la teneur en FvW de multimérisation > 10.

La filtration a été effectuée sur un filtre de même nature (cuprophane, Planova) mais de porosité différente (20 à 22 nm), une solution de FVIII obtenue comme dans l'exemple 1 est ajustée à pH 6 et additionnée de CaCl2 à 0.45 M. La pression est réglée à 17 mbar et l'ensemble solution et filtre est thermostaté à 35 °C.
Dans ces conditions, le débit est de 1.21/h par m² et le rendement de FVIII est de 80 % par rapport au FVIII initial.
Le tableau II donne la composition des multimères de FvW.

**Tableau II: Répartition des multimères de FvW (Planova P21).**

| **Facteur vW** | **Avant filtre** | **Après filtre** |
|---|---|---|
| < pentamères | 47 % | 56 % |
| 5 à 9 mères | 32 % | 34 % |
| 10 à 15 mères | 13 % | **10 %** |
| 16 mères et + | 11 % | **2 %** |

Les décamères-pentadécamères sont significativement réduits et passent de 13 % à 10 %.
Les hexadécamères sont drastiquement réduits de 11 % à 2 %.
Le facteur de réduction du titre viral est de 4.3 log.

### Exemple 3 : Filtration à haute pression et à travers une porosité d'environ 20 nm

Dans le but d'examiner la performance du filtre Planova P21 dans des conditions de pression plus élevées et à température ambiante, la solution de FVIII est ajustée à pH 6 et la concentration en CaCl2 est amenée à 0.35 M. La température est de 22 °C et la pression est ajustée à 400 mbar.

Dans ces conditions, la vitesse de filtration atteint 51/h par m2 et le rendement en Facteur VIII est de 64 % par rapport au produit de départ.
Le tableau III donne la composition en multimères de FvW.

**Tableau III: Répartition des multimères de FvW (Planova P21 à pression élevée)**

| Multimères FvW | Avant filtre | Après filtre |
|---|---|---|
| < 5 mères | 44 % | 50 % |
| 5 à 9 mères | 34% | 35 % |
| 10 à 15 mères | 13% | 8% |
| 16 mères et + | 10 % | 7% |

Les décamères-pentadécamères sont réduits de 13 % à 8 %.
Les hexadécamères et plus sont réduits de 10% à 7 %.
Le facteur de réduction du titre viral est de 6.1 log.

### Exemple 4: test sur filtre de type polysulfone 20 nm à haute pression.

Dans le but de valider un autre type de filtre, un essai de filtration de FVIII a été effectué sur un filtre type polysulfone DV20 (Pall). Ce type de filtre admet des pressions plus élevées que les filtres cuprophane. Ainsi l'exemple n° 3 a été mis en place pour évaluer le comportement du filtre cuprophane à pression plus élevée, de façon à recueillir des observations dans des conditions approchantes.

La solution de FVIII est amenée à pH6 en présence de CaCl2 à 0.35 M. La solution et le filtre sont thermostatés à 35°C. La pression nécessaire à la mise en oeuvre du filtre est de 950 mbar. Dans ces conditions, le débit moyen s'établit à 71/h par m2.
Le rendement en Facteur VIII est de 70 % par rapport au FVIII initial.
Le tableau IV donne la composition en multimères de FvW.

**Tableau IV : Répartition des multimères de FvW (DV20 Pall, polysulfone)**

| Multimères FvW | Avant filtre | Après filtre |
|---|---|---|
| < 5 mères | 35 % | 53 % |
| 5 à 9 mères | 38 % | 30 % |
| 10 à 15 mères | 27 % | **12 %** |
| 16 mères et + | 10 % | **6 %** |

Les hexadécamères subissent une réduction drastique de 10 % à 6 %.

Cependant, la réduction du titre viral n'est que de **2.1 log** ce qui est nettement en dessous de la norme fixée par les autorités réglementaires (> 4 log) pour démontrer l'efficacité d'un procédé d'élimination de virus, en vue de réduire la charge virale potentielle d'un produit dérivé du plasma humain.

### Conclusion

Le tableau V reprend la somme des valeurs des multimères à partir du décamère, on constate que : lorsque la somme décamères et multimères supérieurs de FvW est au plus égale à 15 %, la réduction du titre viral est toujours > 4 log.

En revanche, si cette somme dépasse 16 %, la réduction du titre viral est inférieure à 4 log.

Cette corrélation entre multimères d'ordre 10 et plus de FvW et présence de virus est probablement liée à des phénomènes de passage à travers ces filtres selon les conditions de filtration, la porosité, la nature des matériaux filtrants, la texture du filtre, la géométrie des pores. Tous ces paramètres peuvent avoir une influence sur la rétention ou la non- rétention de virus. Les tests appliqués au filtre donnent, après utilisation, une indication de son efficacité, mais c'est seulement dans le cas de l'invention que le produit filtré, le Facteur VIII accompagné de multimères FVIII caractérisés selon leur degré de multimérisation que l'assurance d'une filtration efficace pour la rétention de virus peut être donnée.
L'efficacité de rétention virale > 4 log est alors reliée à la distribution de multimères de FvW dans le filtrat d'au plus 15 % de multimères de degré de multimérisation > 10. Ces données sont résumées dans le tableau V :

**Tableau V : Corrélation entre le facteur de Réduction (Rf) viral et la composition en multimères FvW > 10 mères.**

| | Exemple 1 | | Exemple 2 | | Exemple 3 | | Exemple 4 | |
|---|---|---|---|---|---|---|---|---|
| | Avant | Après | Avant | Après | Avant | Après | Avant | Après |
| Multimères 10-15 | 15 % | 9% | 13 % | 10 % | 13 % | 8% | 27% | 12 % |
| Multimères 16 et + | 11% | 4% | 10% | 2% | 10% | 7% | 10% | 6% |
| **Total** | **26%** | **13%** | **23%** | **12%** | **23%** | **15%** | **37%** | **18%** |
| Facteur de réduction | | | | | | | | |
| viral (log) | | **6.0** | | **4.3** | | **6.1** | | **2.1** |

## Revendications

1. Composition de Facteur VIII d'origine plasmatique sécurisé viralement, **caractérisée en ce qu'**elle est obtenue après filtration à travers un filtre nanométrique d'ouverture nominale de pores de 15±2 nm à 23±2 nm et **en ce qu'**elle comprend du Facteur von Willebrand (FvW) à un taux inférieur ou égal à 15 % de décamères et multimères supérieurs.

2. Composition selon la revendication 1, **caractérisée en ce que** le facteur de réduction du titre d'un virus d'une taille de 27±3 nm est supérieur ou égale à 4 log, de préférence 5 log, avantageusement 6 log comparé à la solution avant filtration.

3. Composition pharmaceutique selon l'une des revendications 1 et 2 se trouvant sous la forme d'une solution injectable par voie intraveineuse, intramusculaire ou sous-cutanée.

4. Utilisation d'une composition selon l'une des revendications 1 à 3 pour préparer un médicament destiné au traitement des maladies liées à la coagulation sanguine, notamment l'hémophilie.
